# EUROPEAN PATENT SPECIFICATION

(11) **EP 0 664 708 B1**
(45) Date of publication and mention of the grant of the patent: **26.08.1998**
(21) Application number: 93907581.8
(22) Date of filing: 15.03.1993
(51) Int. Cl.: A61K 31/70, A61K 31/505, A61K 31/52, A61K 31/44

(54) **METHOD OF TREATING CANCER BY CONJUNCTIVE THERAPY WITH 2'-HALOMETHYLIDENE DERIVATIVES AND AN S-PHASE OR M-PHASE SPECIFIC ANTINEOPLASTIC AGENT**
VERFAHREN ZUR BEHANDLUNG VON KREBS DURCH KOMBINATIONSTHERAPIE MIT 2'-HALOMETHYLIDENDERIVATEN UND EINEM S- ODER M-PHASE SPEZIFISCHEN ANTINEOPLASTISCHEN WIRKSTOFF
PROCEDE DE TRAITEMENT DU CANCER PAR ASSOCIATION THERAPEUTIQUE DE DERIVES DE 2'-HALOMETHYLIDENE ET D'UN AGENT ANTINEOPLASIQUE SPECIFIQUE PAR RAPPORT A LA PHASE S OU M

(30) Priority: 10.04.1992 US 866399
(43) Date of publication of application: 02.08.1995
(73) Proprietor: MERRELL PHARMACEUTICALS INC., Cincinnati Ohio 45215 (US)
(72) Inventor: SUNKARA, Sai P., Cincinnati, OH 45236 (US)
(74) Representative: Macchetta, Francesco
(86) International application number: US9302490
(87) International publication number: WO9320825

(56) References cited:
- PROCEEDINS 83 ANNUAL MEETING OF THE AMERICAN ASSOCIATION FOR CANCER RESEARCH, vol.33, no.3088, March 1992 page 517 SUNKARA P.S. ET AL. 'Synergistic antitumour activity of (E)-2'-fluoromethylene-2'-deoxycytidine (FMDC, MDL 101.731), an inhibitor of ribonucleotide reductase in combination with S-phase-specific drugs'
- PHARMACEUTICAL RESEARCH, vol.4, 4 July 1984 pages 181 - 183 KOBAYASHI SHU ET AL. 'Experimental Combination Chemotherapy with Thymidylate Synthetase and Ribonucleotide Reductase Inhibitors'
- J. Am. Chem. Soc., 1991, McCARTHY et al., p. 7439-7440, Stereospecific Method to E and Z Terminal Fluoro Olefins and its Application to the Synthesis of 2'-Deoxy-2'-Fluoromethylene Nucleosides as Potential Inhibitors of Ribonucleoside Diphosphate Reductase - see entire Reference Chemotherapy of Cancer, 2nd ED, 13 August 1981, pages 76-83.
- Chemotherapy of Cancer, 2nd ED, 13 August 1981, pages 76-83.

## Description

### BACKGROUND OF THE INVENTION

Neoplastic disease states in humans are recognized throughout the world as being serious and oftentimes life-threatening conditions. These neoplastic diseases, which are characterized by rapidly-proliferating cell growth, have been and continue to be the subject of worldwide research efforts directed toward the identification of therapeutic agents which are effective in the treatment of patients suffering therefrom. Effective therapeutic agents can be characterized as those which prolong the survivability of the patient, which inhibit the rapidly-proliferating cell growth associated with the neoplasm, or which effect a regression of the neoplasm. Research in this area is primarily focused toward identifying agents which would be therapeutically effective in humans. Typically, compounds are tested for antineoplastic activity in small mammals, such as mice, in experiments designed to be predictive of antineoplastic activity not only in those animals but also in humans against specific neoplastic disease states.

Certain S-phase specific antimetabolites and M-phase specific vinca alkaloids are well known as effective antineoplastic agents (See Corr, R.T., and Fritz, W.L., "CANCER CHEMOTHERAPY HANDBOOK", 1980_{,} Elsevier North Holland, Inc., New York; Calabresi, P., and Chabner, B.A., "CHEMOTHERAPY OF NEOPLASTIC DISEASES", Section XII , GOODMAN AND GILLMAN'S THE PHARMACOLOGICAL BASIS OF THERAPEUTICS, 8th ed., 1990, Pergamon Press Inc., Elmsford, New York, and CHEMOTHERAPY AND CANCER, 2nd edition, 1981, pages 76-83).

Cytarabine (ARA-C), fluorouracil (5-FU), mercaptopurine (6-MP), methotrexate (MTX), thioguanine (6-TG), hydroxyurea, prednisone, procarbazine and diglycoaldehyde are examples of antimetabolites with antineoplastic properties. Vincristine and vinblastine are examples of vinca alkaloids with antineoplastic properties. These agents are proven to be useful in the treatment of patients suffering from a variety of neoplastic disease states.

Certain 2'-halomethylidene derivatives of the formula (1) 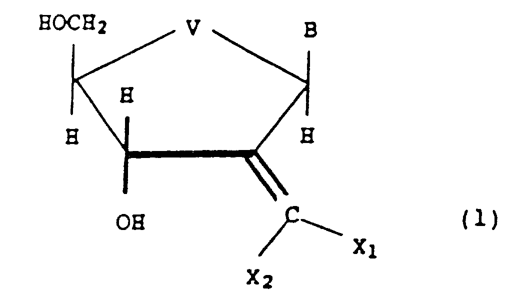 wherein
V is oxy, methylene, or thio,
X₁ and X₂ are each independently hydrogen or halogen,
with the proviso that at least one of X₁ and X₂ is halogen,
B is a radical of the formula 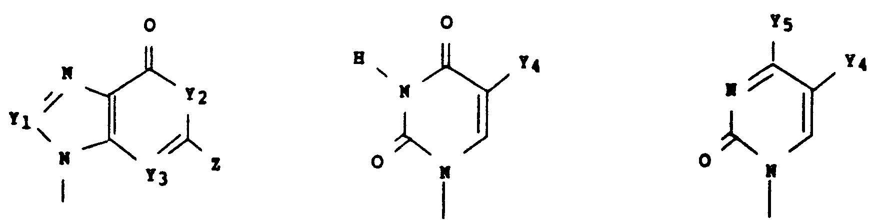
wherein Y₁ is nitrogen, a CH group, a CCl group, a CBr group or a CNH₂ group; Y₂ and Y₃ are each independently nitrogen or a CH group; Y₄ is hydrogen, C₁-C₄ alkyl, C₁-C₄ alkoxy or halogen; Y₅ is amino or C₁-C₄ alkoxy; and Z is hydrogen, halogen, or NH₂;
or a pharmaceutically acceptable salt thereof, such as (E)-2'-deoxy-2'-fluoromethylidenecytidine, are also well known as effective antineoplastic agents [European Patent Application Publication No. 0 372 268, published June 13, 1990]. These 2'-halomethylidene derivatives of formula (1) are ribonucleotide reductase inhibitors with potent antiproliferative and antitumor activity which are also useful in the treatment of patients suffering from a variety of neoplastic disease states.

It has now been found that in treating a patient afflicted with certain neoplastic disease states, conjunctive therapy with a 2'-halomethylidene derivative of formula (1), such as (E)-2'-deoxy-2'-fluoromethylidenecytidine, and a S-phase specific antineoplastic antimetabolite or M-phase specific vinca alkaloid will provide a synergistic antineoplastic effect.

### SUMMARY OF THE INVENTION

The present invention provides a method of treating a patient suffering from a neoplastic disease state comprising administering to said patient an effective antineoplastic amount of a 2'-halomethylidene derivative of formula (1) in conjunctive therapy with an effective antineoplastic amount of a S-phase or M-phase specific antineoplastic antimetabolite or vinca alkaloid.

More specifically, the present invention provides a method of treating a patient suffering from a neoplastic disease state comprising administering an effective antineoplastic amount of 2'-halomethylidene derivative of formula (1) in conjunctive therapy with an effective antineoplastic amount of cytarabine (ARA-C), fluorouracil (5-FU), mercaptopurine (6-MP), methotrexate (MTX), thioguanine (6-TG), hydroxyurea, prednisone, procarbazine, diglycoaldehyde, vincristine or vinblastine.

### DETAILED DESCRIPTION OF THE INVENTION

As used herein, the term "halogen" or "halo-" refers to a fluorine, chlorine, bromine, or iodine atom and the term "nitrogen" refers to a trivalent nitrogen atom attached to two radicals. The term "C₁-C₄ alkyl" refers to a saturated straight or branched chain hydrocarbyl radical of one to four carbon atoms and includes methyl, ethyl, propyl, isopropyl, n-butyl, isobutyl, tertiary-butyl and the like. The term "C₁-C₄ alkoxy" refers to a C₁-C₄ alkyl bearing an oxy group and includes methoxy, ethoxy, propoxy, butoxy and the like.

As used herein, the term "S-phase specific agent" refers to those agents which exert their cytotoxic activity in the S-phase or DNA synthetic phase of the cell cycle. Included within the meaning of the term are agents which exert their cytotoxic effect during the S-phase of the cell cycle and subsequently result in cell death. Examples of cytotoxic agents in this class are the antimetabolites cytarabine (ARA-C), fluorouracil (5-FU), mercaptopurine (6-MP), methotrexate (MTX), thioguanine (6-TG), hydroxyurea, prednisone, procarbazine and diglycoaldehyde. Also included within the meaning of the term "S-phase specific agent" are agents which exert their cytotoxic effect during the S phase but result in cell death while the cell is in the M phase or mitosis phase of the cell cycle. Examples of cytotoxic agents in this class are the nitrosoureas and bleomycin.

As used herein, the term "M-phase specific agent" refers to those agents which exert their cytotoxic activity in the M-phase or mytotic phase of the cell cycle. Included within the meaning of the term are agents which exert their Cytotoxic effect during the M-phase of the cell cycle and subsequently result in cell death. Examples of cytotoxic agents in this class are the etoposide, vincristine and vinblastine.

2'-Halomethylidene derivatives of formula (1) can be prepared as described in European Patent Application Publication No. 0 372 268, published June 13, 1990. In order to illustrate the preparation of the 2'-halomethylidene derivatives of formula (1), the following example illustrating the preparation of (E)-2'-deoxy-2'-fluoromethylidenecytidine is provided. The example is illustrative only and is not intended to limit the invention in any way. All temperatures are in degrees Celsius and the following abbreviations are used: (g) is grams, (mol) is moles, (ml) is milliliters, (l) is liters, (lb/in²) is pounds per square inch, (TLC) is thin layer chromatography, (THF) is tetrahydrofuran, (DMF) is dimethylformamide, (mp) is melting point, (mm/Hg) is pressure expressed as millimeters of mercury, and (bp) is boiling point.

### EXAMPLE 1

### (Z)- and (E)-2'-DEOXY-2'-FLUOROMETHYLIDENECYTIDINE Step a: 4-Ethoxy-1-[(3,5-O-tetraisopropyldisiloxan-1,3-diyl)-β-D-erythro-pentofuran-2-(2-fluoro-2-phenylsulfonyl methylidene)osyl]-2(1H)-pyrimidone

Prepare diethylfluoromethylphenylsulfonylphosphonate as follows: To a solution of fluoromethylphenyl sulfone (500 mg, 2.87 mmol) in dry THF (30 ml) which has been cooled to about -60°C in a dry 3-necked 100 ml flask with stirring bar, argon inlet valve, thermometer and rubber septum, add diethyl chlorophosphate (500 mg, 0.42 ml, 2.87 mmol) via syringe. To this mixture, add a solution of 1.65 M lithium diisopropylamide in cyclohexane (3.48 ml, 5.74 mmol) via syringe and follow the formation of diethylfluoromethylphenylsulfonylphosphonate by gas-liquid chromatography (GLC).

To the diethylfluoromethylphenylsulfonylphosphonate solution above add a solution of 4-ethoxy-1-[(3,5-O-tetraisopropyldisiloxan-1,3-diyl)-2-keto-β-D-erythropentofuranosyl]-2(1H)-pyrimidone (732 mg, 2 mmol) in dry THF (about 5 ml) and allow the reaction mixture to warm to room temperature overnight under an argon atmosphere. Pour the mixture into a saturated, ice-cold solution of ammonium chloride and extract the mixture with ethyl acetate (3 times, 75 ml each time). Combine the organic layers, dry with anhydrous magnesium sulfate, and evaporate to dryness. Chromatograph the residue on a silica gel flash column eluting with ethyl acetate/hexane (1/1, v/v) to provide the title compound.

### Step b: 4-Ethoxy-1-[β-D-erythro-pentofuran-2-(2-fluoro-2-phenylsulfonylmethylidene)osyl]-2(1H)-pyrimidone

To a solution of 1.0 M tetrabutylammonium fluoride in THF (2.2 ml, 2.2 mmol) add 4-ethoxy-1-[(3,5-O-tetraisopropyldisiloxan-1,3-diyl)-β-D-erythro-pentofuran-2-(2-fluoro-2-phenylsulfonylmethylidene)osyl]-2(1H)-pyrimidone (668 mg, 1 mmol) and stir the mixture at room temperature for 2 hours. Neutralize the mixture with acetic acid, add flash silica gel to the mixture and evaporate to dryness *in vacuo*. Apply the residue to a flash silica gel column and elute with chloroform/ethanol (20/1, v/v) to provide the title compound.

### Step c: (Z)- and (E)-2'-Deoxy-2'-fluoromethylidenecytidine

To a solution of 4-ethoxy-1-[β-D-erythio-pentofuran-2-(2-fluoro-2-phenylsulfonylmethylidene)osyl]-2(1H)-pyrimidone (854 mg, 2 mmol) in 10% aqueous THF (100 ml) under a nitrogen atmosphere, add aluminum amalgam (made from 0.04 g aluminum in 2% aqueous HgCl₂). Stir and vent the mixture while refluxing for 2 hours. Filter the mixture and evaporate most of the THF *in vacuo*. Extract the residue with ethyl acetate (3 times, 25 ml each time), combine the organic layers and dry with anhydrous Na₂SO₄. Evaporate to dryness *in vacuo* and apply the residue to a flash silica gel column and elute with chloroform/ethanol (9/1, v/v) to provide (Z)- and (E)-4-ethoxy-1-[β-D-erythro-pentofuran-2-(2-fluoromethylidene)osyl]-2(1H)-pyrimidone as a mixture of geometric isomers.

Heat a solution of (Z)- and (E)-4-ethoxy-1-[β-D-erythro-pentofuran-2-(2-fluoromethylidene)osyl]-2(1H)-pyrimidone (858 mg, 3 mmol) in methanolic ammonia (10 ml, saturated at 0°C) in a sealed tube at 100°C for 2 days. Evaporate the solution to dryness and separate the (Z) and (E) isomers of the title compound by chromatography by applying the residue to a column packed with Dowex l-X2 (OH⁻ form) and eluting with methanol.

The antineoplastic antimetabolites, such as cytarabine (ARA-C), fluorouracil (5-FU), mercaptopurine (6-MP), methotrexate (MTX), thioguanine (6-TG), hydroxyurea, prednisone, procarbazine and diglycoaldehyde and the antineoplastic vinca alkaloids, such as vincristine and vinblastine, are readily available and their use as antineoplastic agents is well known and appreciated in the art [For example, See Corr, R. T., and Fritz, W. L., "CANCER CHEMOTHERAPY HANDBOOK", 1980, Elsevier North Holland, Inc., New York, New York and Calabresi, P., and Chabner, B. A., "CHEMOTHERAPY OF NEOPLASTIC DISEASES", Section XII, GOODMAN AND GILLMAN'S THE PHARMACOLOGICAL BASIS OF THERAPEUTICS, 8th ed., 1990, Pergamon Press Inc., Elmsford, New York].

The present invention provides a method of treating a patient suffering from a neoplastic disease state comprising conjunctive therapy with an effective antineoplastic amount of a 2'-halomethylidene derivative of formula (1) and an effective antineoplastic amount of a S-phase specific antineoplastic antimetabolite or M-phase specific vinca alkaloid. This conjunctive therapy unexpectedly provides a synergistic antineoplastic effect.

As used herein, the term "patient" refers to a warm-blooded animal such as a mammal which is afflicted with a neoplastic disease state. It is understood that dogs, cats, rats, mice, horses, bovine cattle, sheep, and humans are examples of animals within the scope of the meaning of the term.

The term "neoplastic disease state" as used herein refers to an abnormal state or condition characterized by rapidly proliferating cell growth or neoplasm. Neoplastic disease states for which conjunctive therapy according to the present invention will be particularly useful include: Leukemias such as, but not limited to, acute lymphoblastic, chronic lymphocytic, acute myeloblastic and chronic myelocytic Carcinomas, such as, but not limited to, those of the cervix, oesophagus, stomach, small intestines, brain, colon and lungs; Sarcomas, such as, but riot limited to, osteoma, osteosarcoma, lipoma, liposarcoma, hemangioma and hemangiosarcoma; Melanomas, including amelanotic and melanotic; and mixed types of neoplasias such as, but not limited to carcinosarcoma, lymphoid tissue type, follicular reticulum, cell sarcoma and Hodgkin's disease. Of course, one skilled in the art will recognize that not every combination of conjunctive therapy according to the present invention will be equally effective against each of the neoplastic disease states. Selection of the most appropriate combination is within the ability of one of ordinary skill in the art and will depend on a variety of factors including assessment of results obtained in standard animal cancer models and the effectiveness of the individual agents as monotherapy in treating particular neoplastic disease states.

For example, conjunctive therapy with a 2'-halomethylidene derivative of formula (1) and cytarabine will be particularly effective in the treatment of a patient afflicted with acute granulocytic and acute lymphocytic leukemias. Conjunctive therapy with a 2'-halomethylidene derivative of formula (1) and hydroxyurea will be particularly effective in the treatment of a patient afflicted with chronic granulocytic leukemia, polycythemia vera, essential thrombocytosis and malignant melanoma. Conjunctive therapy with a 2'-halomethylidene derivative of formula (1) and prednisone will be particularly effective in the treatment of a patient afflicted with acute and chronic lymphocytic leukemias, non-Hodgkin's lymphomas, Hodgkin's disease and breast carcinoma. Conjunctive therapy with a 2'-halomethylidene derivative of formula (1) and methotrexate will be particularly effective in the treatment of a patient afflicted with acute lymphocytic leukemia, choriocarcinoma, mycosis fungoides, breast, head and neck carcinoma, and lung osteogenic sarcoma. Conjunctive therapy with a 2'-halomethylidene derivative of formula (1) and fluorouracil will be particularly effective in the treatment of a patient afflicted with breast, colon, stomach, pancreas, ovary, head and neck carcinoma, urinary bladder carcinoma and premalignant skin lesions. Conjunctive therapy with a 2'-halomethylidene derivative of formula (1) and mercaptopurine will be particularly effective in the treatment of a patient afflicted with acute lymphocytic, acute granulocytic and chronic granulocytic leukemias. Conjunctive therapy with a 2'-halomethylidene derivative of formula (1) and thioguanine will be particularly effective in the treatment of a patient afflicted with acute granulocytic, acute lymphocytic, and chronic granulocytic leukemias. Conjunctive therapy with a 2'-halomethylidene derivative of formula (1) and procarbazine will be particularly effective in the treatment of a patient afflicted with Hodgkin's disease. Conjunctive therapy with a 2'-halomethylidene derivative of formula (1) and vincristine will be particularly effective in the treatment of a patient afflicted with acute lymphocytic leukemia, neuroblastoma, Wilms' tumor, rhabdomyosarcoma, Hodgkin's disease and small-cell lung carcinoma. Conjunctive therapy with a 2'-halomethylidene derivative of formula (1) and vinblastine will be particularly effective in the treatment of a patient afflicted with Hodgkin's disease, non-Hodgkin's lymphomas, breast and testis carcinoma.

In effecting treatment of a patient afflicted with a neoplastic disease state as described above, a 2'-halomethylidene derivative of formula (1) is administered in conjunctive therapy with a S-phase specific antineoplastic antimetabolite or M-phase specific vinca alkaloid. As used herein, the term "conjunctive therapy" contemplates co-administration of a 2'-halomethylidene derivative of formula (1) along with the S-phase specific antineoplastic antimetabolite or M-phase specific vinca alkaloid. This co-administration may take place at essentially the same time, it may take place sequentially, or it may take place alternately.

In providing co-administration at essentially the same time, the courses of treatment with a 2'-halomethylidene derivative of formula (1) and the selected S-phase specific antineoplastic antimetabolite or M-phase specific vinca alkaloid run essentially concomitantly. In providing sequential co-administration, a full course of treatment of one of the agents is terminated and then followed by a full course of treatment of the other. In providing alternate co-administration, a partial course of treatment of one of the agents is terminated and then followed by a partial course of treatment of the other in an alternating manner until a full treatment of each agent is administered. When the 2'-halomethylidene derivative of formula (1) and the selected S-phase specific antineoplastic antimetabolite or M-phase specific vinca alkaloid are co-administered in a sequential or an alternate manner, it is generally preferred to administer the 2'-halomethylidene derivative of formula (1) first and the S-phase specific antineoplastic antimetabolite or M-phase specific vinca alkaloid last.

In effecting the conjunctive therapy according to the present invention, it is preferred to co-administer the 2'-halomethylidene derivative of formula (1) and the selected S-phase specific antineoplastic antimetabolite or M-phase specific vinca alkaloid in a sequential or an alternate manner. It is most preferred to co-administer the 2'-halomethylidene derivative of formula (1) and the selected S-phase specific antineoplastic antimetabolite or M-phase specific vinca alkaloid in a sequential manner.

As used herein, the term "effective antineoplastic amount" refers to an amount which is effective, upon single or multiple dose administration to the patient, in controlling the growth of the neoplasm or in prolonging the survivability of the patient beyond that expected in the absence of such treatment. As used herein, "controlling the growth" of the neoplasm refers to slowing, interrupting, arresting or stopping its growth and does not necessarily indicate a total elimination of the neoplasm.

An effective antineoplastic amount of a 2'-halomethylidene derivative of formula (1) is expected to vary from about 10 milligram per kilogram of body weight per day (mg/kg/day) to about 100 mg/kg/day and preferably will be about 5 mg/kg/day to about 50 mg/kg/day.

The effective antineoplastic amounts of the various S-phase specific antineoplastic antimetabolites and M-phase specific vinca alkaloids are well known and appreciated in the art. For example, an effective antineoplastic amount of cytarabine (ARA-C) is expected to vary from about 1 mg/m²/day to about 200 mg/m²/day. An effective antineoplastic amount of fluorouracil (5-FU) is expected to vary from about 6 mg/m²/day to about 800 mg/m²/day. An effective antineoplastic amount of mercaptopurine (6-MP) is expected to vary from about 2.5 mg/m²/day to about 700 mg/m²/day. An effective antineoplastic amount of methotrexate (MTX) is expected to vary from about 2.5 mg/m²/day to about 30 mg/m²/day. An effective antineoplastic amount of thioguanine (6-TG) is expected to vary from about 2 mg/kg/day to about 3 mg/kg/day. An effective antineoplastic amount of hydroxyurea is expected to vary from about 20 mg/kg/day to about 80 mg/kg/day. An effective antineoplastic amount of prednisone is expected to vary from about 15 mg/m²/day to about 100 mg/m²/day. An effective antineoplastic amount of procarbazine is expected to vary from about 1 mg/kg/day to about 4 mg/kg/day. An effective antineoplastic amount of diglycoaldehyde is expected to vary from about 0.25 mg/kg/day to about 5 mg/kg/day. An effective antineoplastic amount of vincristine is expected to vary from about 0.4 mg/m²/week to about 2 mg/m²/week. An effective antineoplastic amount of vinblastine is expected to vary from about 4.0 mg/m²/week to about 2 mg/m²/day.

In effecting treatment of a patient afflicted with a disease state described above, the 2'-halomethylidene derivatives of formula (1) can be administered in any form or mode which makes the compound bioavailable in effective amounts, including oral and parenteral routes. For example, it can be administered orally, subcutaneously, intramuscularly, intravenously, transdermally, intranasally, rectally, and the like. Oral administration is generally preferred. One skilled in the art of preparing formulations can readily select the proper form and mode of administration depending upon the particular circumstances, including the disease state to be treated, the stage of the disease, the form of administration of the selected S-phase specific antineoplastic antimetabolite or vinca alkaloid, the manner of co-administration selected, and the like.

In effecting treatment of a patient afflicted with a disease state described above, the 2'-halomethylidene derivatives of formula (1) can be administered in combination with the various S-phase specific antineoplastic antimetabolites or M-phase specific vinca alkaloids in the proportions of antineoplastic amount of a 2'-halomethylidene derivative of formula (1) to antineoplastic amount of a S-phase or M-phase specific agent in the range of about 1:0.1 to about 1:50, more preferably in the range of about 1:1 to about 1:20 and most preferably in the range of about 1:1 to about 1:10.

The 2'-halomethylidene derivatives of formula (1) can be administered alone or in the form of a pharmaceutical composition in combination with pharmaceutically acceptable carriers or excipients, the proportion and nature of which are determined by the solubility and chemical properties of the 2'-halomethylidene derivatives of formula (1), the chosen route of administration, and standard pharmaceutical practice. The 2'-halomethylidene derivative of formula (1), while effective itself, may be formulated and administered in the form of its pharmaceutically acceptable acid addition salt for purposes of stability, convenience of crystallization, increased solubility and the like.

The selected S-phase specific antineoplastic antimetabolite or M-phase specific vinca alkaloid can be administered in a manner as is well known and accepted for the particular agent. For example, cytarabine, fluorouracil, methotrexate, thioguanine, hydroxyurea, procarbazine, mercaptopurine (as its sodium salt), vinblastine and vincristine may be administered intravenously. Mercaptopurine, methotrexate, thioguanine, hydroxyurea, prednisone, procarbazine and diglycoaldehyde may be administered orally. Methotrexate may also be administered via intramuscular, intraarterial and intrathecal routes.

The following examples are provided in order to illustrate the method of use of the present invention. These examples are intended to be illustrative only and are not to be construed to limit the scope of the invention in any way.

### Example 2

### Synergistic Antitumor Activity of A Combination of MDL 101,731 and ARA-C Against B16 Melanoma in Mice

Inject B16 melanoma cells (1X10⁵ cells/mouse) subcutaneously on day 0. Administer compounds i.p. once daily as indicated. Sacrifice the animals on day 15, dissect the tumors and weigh.

**Table 1**

| Antitumor Activity against B16 Melanoma | | |
|---|---|---|
| Treatment | Tumor Weight (g) (Mean ± S.D.^{a}) | % Inhibition |
| Control | 2.6 ± 0.5 | - |
| 101,731^{b} (5 mg/kg, day 1-7) | 2.4 ± 0.9 | 10 |
| ARA-C (10 mg/kg, day 7-14) | 3.6 ± 0.8 | 0 |
| 101,731 + ARA-C | 1.0 ± 0.3 | 59 |

| | | |
|---|---|---|
| ^{a} S.D. = Standard Deviation | | |
| ^{b}101,731 = (E)-2'-deoxy-2'-fluoromethylidenecytidine | | |

### Example 3

### Synergistic Antitumor Activity of A Combination of MDL 101,731 and ARA-C Against Lewis Lung Carcinoma in Mice

Inject 3LL cells (1X10⁵/mouse) s.c. on day 0. Administer compounds i.p., once daily as indicated. Sacrifice animals on day 15, dissect the tumors and weigh. Also count pulmonary metastatic foci.

**Table 2**

| Antitumor Activity against Lewis Lung Carcinoma | | | | |
|---|---|---|---|---|
| Treatment | Tumor Weight (g) (Mean ± S.D.^{a}) | % Inhibition | No. of Foci (Mean ± S.D.^{a}) | % Inhibition |
| Control | 3.5 ± 0.4 | - | 11.3 ± 4 | - |
| 101,731^{b} (5 mg/kg, day 1-7) | 2.8 ± 0.6 | 20 | 15.5 ± 6 | 0 |
| ARA-C (10 mg/kg, day 7-14) | 3.83 ± 0.8 | 0 | 14.6 ± 5 | 0 |
| 101,731 + ARA-C | 1.12 ± 0.3 | 69 | 0 | 100 |

| | | | | |
|---|---|---|---|---|
| ^{a} S.D. = Standard Deviation | | | | |
| ^{b} 101,731 = (E)-2'-deoxy-2'-fluoromethylidenecytidine | | | | |

### Example 4

### Synergistic Antiproliferative Activity of MDL 101,731 in Combination with S or M Phase Specific Antitumor Agents Against HeLa Cells

Plate HeLa cells (2X10³ cells/well) and allow to grow for 18 hours. Treat with MDL 101,731 (15ng/mL) for 24 hours. Wash the compound and expose cells to the indicated drugs for another 72 hours. Determine the cell viability by a colorimetric assay, essentially as described by Carmichael et al. [*Cancer Res*. 47, 936 (1987)], whereby the cellular reduction of MTT [3-(4,5-dimethylthiazol-2-yl)-2,5-diphenyl-2H-tetrazolium bromide] is measured. Treatment with MDL 101,731 alone as described did not show any effect on the growth of the tumor cells.

Calculate IC₅₀ values for the individual treatments as well as for the combined treatments of MDL 101,731 with the various other agents. The IC₅₀ values at the various concentrations of test agents are presented in Table 3.

**Table 3**

| Antiproliferative Activity of MDL 101,731 in Combination with S or M Phase Specific Anti-tumor Agents Against HeLa Cells | | |
|---|---|---|
| Drug | Drug Alone IC₅₀(µg/mL) | Drug + 101,731^{a} IC₅₀(µg/mL) |
| Cytosine Arabinoside (ARA-C) | 13.7 | 3.2 |
| 5-Fluorouracil (5-FU) | 41.1 | 18.9 |
| Vinblastine (VLB) | 86.7 | 13.6 |

| | | |
|---|---|---|
| ^{a} 101,731 = (E)-2'-deoxy-2'-fluoromethylidenecytidine | | |

## Claims

1. A combination, comprising a 2'-halomethylidene derivative of the formula (1) 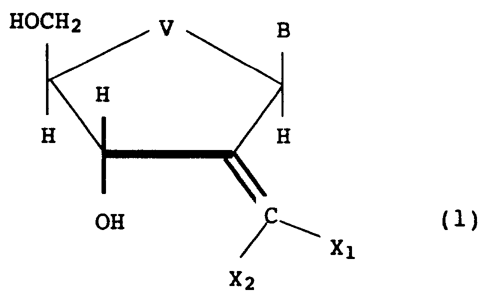 wherein
V is oxy, methylene, or thio,
X₁ and X₂ are each independently hydrogen or halogen,
with the proviso that at least one of X₁ and X₂ is halogen,
B is a group of the formula 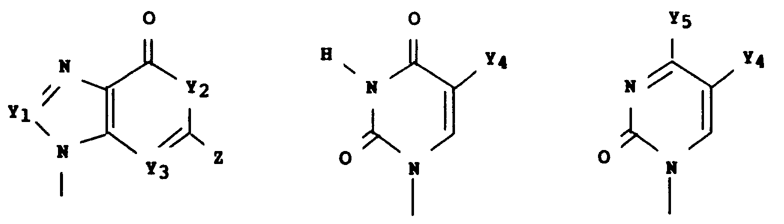
wherein Y₁ is nitrogen, a CH group, a CCl group, a CBr group or a CNH₂ group; Y₂ and Y₃ are each independently nitrogen or a CH group; Y₄ is hydrogen, C₁-C₄ alkyl, C₁-C₄ alkoxy or halogen; Y₅ is amino or C₁-C₄ alkoxy; and Z is hydrogen, halogen, or NH₂;
or a pharmaceutically acceptable salt thereof and a S-phase or M-phase specific agent, for the conjunctive treatment of a neoplastic disease state.

2. A combination according to Claim 1 wherein the 2'-halomethylidene derivative of formula (1) is (E)-2'-deoxy-2'-fluoromethylidenecytidine.

3. A combination according to Claim 1 or 2 wherein the S-phase specific agent is cytarabine.

4. A combination according to Claim 1 or 2 wherein the S-phase specific agent is fluorouracil.

5. A combination according to Claim 1 or 2 wherein the M-phase specific agent is vinblastine.

6. A combination according to anyone of Claims 1 to 5 wherein the neoplastic disease state is a leukemia.

7. A combination according to anyone of Claims 1 to 5 wherein the neoplastic disease state is a carcinoma.

8. Use of a compound of the formula (1) 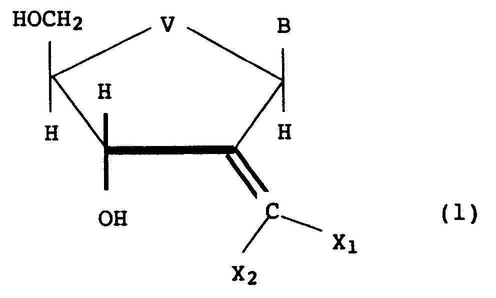 wherein
V is oxy, methylene, or thio,
X₁ and X₂ are each independently hydrogen or halogen,
with the proviso that at least one of X₁ and X₂ is halogen,
B is a group of the formula 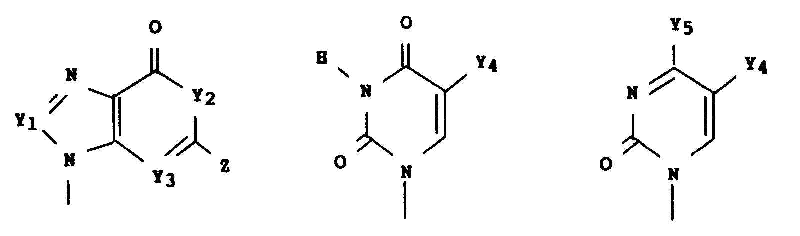
wherein Y₁ is nitrogen, a CH group, a CCl group, a CBr group or a CNH₂ group; Y₂ and Y₃ are each independently nitrogen or a CH group; Y₄ is hydrogen, C₁-C₄ alkyl, C₁-C₄ alkoxy or halogen; Y₅ is amino or C₁-C₄ alkoxy; and Z is hydrogen, halogen, or NH₂;
or a pharmaceutically acceptable salt thereof for the preparation of a medicament for the treatment of a neoplastic disease state in a conjunctive therapy with an antineoplastic S-phase or M-phase specific agent.

9. A use according to claim 8 wherein the 2'-halomethylidene derivative of formula (1) is (E)-2'-deoxy-2'-fluoromethylidenecytidine.

10. A use according to Claim 8 or 9 wherein the S-phase specific agent is cytarabine.

11. A use according to Claim 8 or 9 wherein the S-phase specific agent is fluorouracil.

12. A use according to Claim 8 or 9 wherein the M-phase specific agent is vinblastine.

13. A use according to anyone of Claims 9 to 12 wherein the neoplastic disease state is a leukemia.

14. A use according to anyone of Claims 9 to 12 wherein the neoplastic disease state is a carcinoma.

## Patentansprüche

1. Kombination, die ein 2'-Halogenmethylidenderivat der Formel (1) 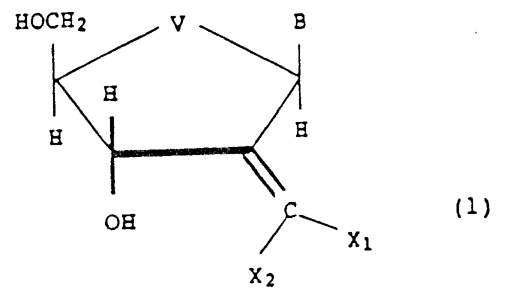 in der
V eine Oxy-, Methylen- oder Thiogruppe bedeutet,
X₁ und X₂ jeweils unabhängig voneinander ein Wasserstoff- oder Halogenatom darstellen,
mit der Maßgabe, daß mindestens einer der Reste X₁ und X₂ ein Halogenatom bedeutet,
B einen Rest der Formel 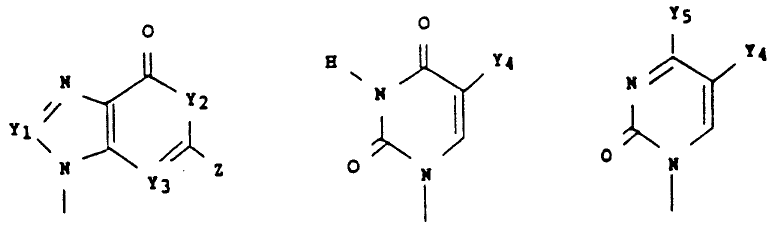
darstellt, in der Y₁ ein Stickstoffatom, eine CH-Gruppe, eine CCl-Gruppe, eine CBr-Gruppe oder eine CNH₂-Gruppe bedeutet, Y₂ und Y₃ jeweils unabhängig voneinander ein Stickstoffatom oder eine CH-Gruppe darstellen, Y₄ ein Wasserstoffatom, einen C₁-C₄-Alkyl- oder C₁-C₄-Alkoxyrest oder ein Halogenatom bedeuten, Y₅ eine Aminogruppe oder einen C₁-C₄-Alkoxyrest darstellt und Z ein Wasserstoff- oder Halogenatom oder eine NH₂-Gruppe bedeutet,
oder ein pharmazeutisch verträgliches Salz davon und einen S-phasen- oder M-phasenspezifischen Wirkstoff für die Kombinationsbehandlung eines neoplastischen Erkrankungszustandes umfaßt.

2. Kombination nach Anspruch 1, in der das 2'-Halogenmethylidenderivat der Formel (1) (E)-2'-Desoxy-2'-fluormethylidencytidin ist.

3. Kombination nach Anspruch 1 oder 2, in der der S-phasenspezifische Wirkstoff Cytarabin ist.

4. Kombination nach Anspruch 1 oder 2, in der der S-phasenspezifische Wirkstoff Fluoruracil ist.

5. Kombination nach Anspruch 1 oder 2, in der der M-phasenspezifische Wirkstoff Vinblastin ist.

6. Kombination nach einem der Ansprüche 1 bis 5, wobei der neoplastische Erkrankungszustand eine Leukämie ist.

7. Kombination nach einem der Ansprüche 1 bis 5, wobei der neoplastische Erkrankungszustand ein Karzinom ist.

8. Verwendung einer Verbindung der Formel (1) 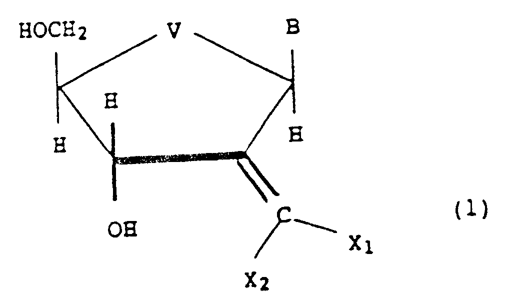 in der
V eine Oxy-, Methylen- oder Thiogruppe bedeutet,
X₁ und X₂ jeweils unabhängig voneinander ein Wasserstoff- oder Halogenatom darstellen,
mit der Maßgabe, daß mindestens einer der Reste X₁ und X₂ ein Halogenatom bedeutet,
B einen Rest der Formel 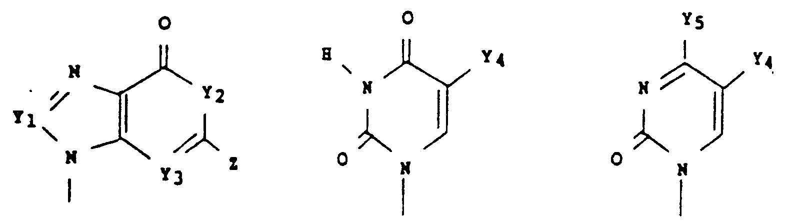
darstellt, in der Y₁ ein Stickstoffatom, eine CH-Gruppe, eine CCl-Gruppe, eine CBr-Gruppe oder eine CNH₂-Gruppe bedeutet, Y₂ und Y₃ jeweils unabhängig voneinander ein Stickstoffatom oder eine CH-Gruppe darstellen Y₄ ein Wasserstoffatom, einen C₁-C₄-Alkyl- oder C₁-C₄-Alkoxyrest oder ein Halogenatom bedeuten, Y₅ eine Aminogruppe oder einen C₁-C₄-Alkoxyrest darstellt und Z ein Wasserstoff- oder Halogenatom oder eine NH₂-Gruppe bedeutet,
oder ein pharmazeutisch verträgliches Salz davon zur Herstellung eines Arzneimittels zur Behandlung eines neoplastischen Erkrankungszustandes in einer Kombinationstherapie mit einem antineoplastischen S-phasen- oder M-phasenspezifischen Wirkstoff.

9. Verwendung nach Anspruch 8, wobei das 2'-Halogenmethylidenderivat der Formel (1) (E)-2'-Desoxy-2'-fluormethylidencytidin ist.

10. Verwendung nach Anspruch 8 oder 9, wobei der S-phasenspezifische Wirkstoff Cytarabin ist.

11. Verwendung nach Anspruch 8 oder 9, wobei der S-phasenspezifische Wirkstoff Fluoruracil ist.

12. Verwendung nach Anspruch 8 oder 9, wobei der M-phasenspezifische Wirkstoff Vinblastin ist.

13. Verwendung nach einem der Ansprüche 9 bis 12, wobei der neoplastische Erkrankungszustand eine Leukämie ist.

14. Verwendung nach einem der Ansprüche 9 bis 12, wobei der neoplastische Erkrankungszustand ein Karzinom ist.

## Revendications

1. Association, comprenant un dérivé de 2'-halogénométhylidène de formule (1) 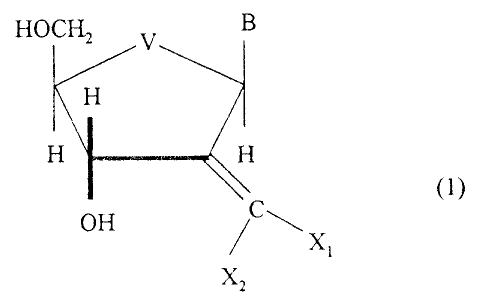 où
V est un groupe oxy, méthylène ou thio,
X₁ et X₂ sont chacun indépendamment un atome d'hydrogène ou d'halogène,
pour autant qu'au moins X₁ ou X₂ soit un halogène,
B est un groupe de formule 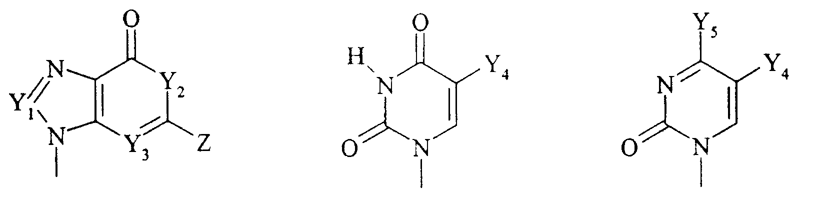
où Y₁ est un atome d'azote, un groupe CH, un groupe CCl, un groupe CBr ou un groupe CNH₂ ; Y₂ et Y₃ sont chacun indépendamment un atome d'azote ou un groupe CH ; Y₄ est un atome d'hydrogène, un groupe alkyle en C₁-C₄, alcoxy en C₁-C₄ ou un atome d'halogène ; Y₅ est un groupe amine ou alcoxy en C₁-C₄ ; et Z est un atome d'hydrogène, un atome d'halogène ou NH₂ ;
ou un sel pharmaceutiquement acceptable de ce dernier, et un agent spécifique de la phase S ou de la phase M, pour le traitement par association d'un tableau clinique néoplasique.

2. Association selon la revendication 1, où le dérivé de 2'-halogénométhylidène de formule (1) est la (E)-2'-désoxy-2'-fluorométhylidènecytidine.

3. Association selon la revendication 1 ou 2, l'agent spécifique de la phase S est la cytarabine.

4. Association selon la revendication 1 ou 2, l'agent spécifique de la phase S est le fluoro-uracile.

5. Association selon la revendication 1 ou 2, où l'agent spécifique de la phase M est la vinblastine.

6. Association selon l'une quelconque des revendications 1 à 5, où le tableau clinique néoplasique est une leucémie.

7. Association selon l'une quelconque des revendications 1 à 5, où le tableau clinique néoplasique est un cancer.

8. Utilisation d'un compose de formule (1) 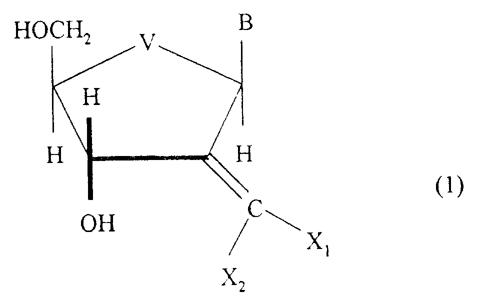 où
V est un groupe oxy, méthylène ou thio,
X₁ et X₂ sont chacun indépendamment un atome d'hydrogène ou d'halogène,
pour autant qu'au moins X₁ ou X₂ soit un atome d'halogène,
B est un groupe de formule 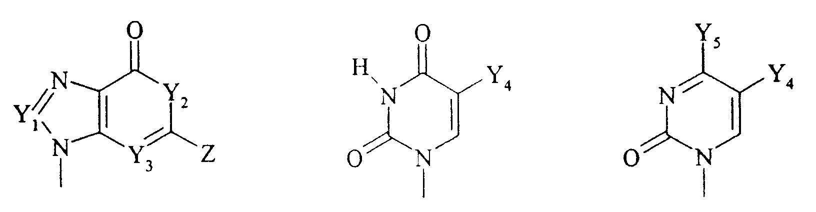
où Y₁ est un atome d'azote, un groupe CH, un groupe CCl, un groupe CBr ou un groupe CNH₂ ; Y₂ et Y₃ sont chacun indépendamment un atome d'azote ou un groupe CH ; Y₄ est un atome d'hydrogène, un groupe alkyle en C₁-C₄, alcoxy en C₁-C₄ ou un atome d'halogène ; Y₅ est un groupe amine ou alcoxy en C₁-C₄ ; et Z est un atome d'hydrogène, un atome d'halogène ou NH₂ ;
ou d'un sel pharmaceutiquement acceptable de ce dernier pour la préparation d'un médicament destiné au traitement d'un tableau clinique néoplasique selon un traitement par association avec un agent antinéoplasique spécifique de la phase S ou de la phase M.

9. Utilisation selon la revendication 8, où le dérivé de 2'-halogénométhylidène de formule (1) est la (E)-2'-désoxy-2'-fluorométhylidènecytidine.

10. Utilisation selon la revendication 8 ou 9, où l'agent spécifique de la phase S est la cytarabine.

11. Utilisation selon la revendication 8 ou 9, où l'agent spécifique de la phase S est le fluoro-uracile.

12. Utilisation selon la revendication 8 ou 9, où l'agent spécifique de la phase M est la vinblastine.

13. Utilisation selon l'une quelconque des revendications 9 à 12, où le tableau clinique néoplasique est une leucémie.

14. Utilisation selon l'une quelconque des revendications 9 à 12, où le tableau clinique néoplasique est un cancer.
